# EUROPÄISCHE PATENTSCHRIFT

(11) **EP 1 635 716 B1**
(45) Veröffentlichungstag und Bekanntmachung des Hinweises auf die Patenterteilung: **11.08.2010**
(21) Anmeldenummer: 04739682.5
(22) Anmeldetag: 08.06.2004
(51) Int. Cl.: A61B 17/28, A61B 17/32, A61B 18/14

(54) **CHIRURGISCHES INSTRUMENT**
SURGICAL INSTRUMENT
INSTRUMENT CHIRURGICAL

(30) Priorität: 20.06.2003 DE 10328516
(43) Veröffentlichungstag der Anmeldung: 22.03.2006
(73) Patentinhaber: Aesculap AG, 78532 Tuttlingen (DE)
(72) Erfinder: KUPFERSCHMID, Bernhard, 78576 Emmingen-Liptingen (DE); MAYENBERGER, Rupert, 78239 Rielasingen (DE); WEISSHAUPT, Dieter, 78194 Immendingen (DE)
(74) Vertreter: Hoeger, Stellrecht & Partner Patentanwälte
(86) Internationale Anmeldenummer: PCT/EP2004/006153
(87) Internationale Veröffentlichungsnummer: WO 2004/112621

(56) Entgegenhaltungen:
- WO-A-01/19261
- DE-U- 20 121 161
- US-A- 5 637 110
- US-A- 5 727 428
- US-A- 6 015 426
- US-A1- 2003 114 839

## Beschreibung

Die vorliegende Erfindung betrifft ein chirurgisches Instrument mit einem eine Längsachse definierenden Schaft, mit einem an einem distalen Ende des Schafts beweglich gelagerten Werkzeug und mit einem im Schaft axial beweglich gelagerten Kraftübertragungsglied zum Übertragen von am proximalen Ende des Instruments auf das Kraftübertragungsglied zum Bewegen des Werkzeugs ausgeübten Zug- und/oder Schubkräften, wobei eine Kupplungsvorrichtung vorgesehen ist zum axialen Verbinden des Kraftübertragungsglieds mit dem mindestens einen Werkzeug in einer Kupplungsstellung, wobei die Kupplungsvorrichtung in der Kupplungsstellung form- und/oder kraftschlüssig mit dem Kraftübertragungsglied verbunden ist, wobei die Kupplungsvorrichtung im Schaft axial geführt ist, wobei eine Verdrehsicherung vorgesehen ist zum Verhindern einer Drehung der Kupplungsvorrichtung um die Längsachse des Schafts und wobei mehrere Kupplungselemente vorgesehen sind.

Chirurgische Instrumente der eingangs beschriebenen Art sind vielfältig bekannt, beispielsweise in Form von endoskopischen Rohrschaftinstrumenten. Dabei ist es erforderlich, das im Schaft beweglich gelagerte Kraftübertragungsglied, welches häufig als Schub- und Zugstange ausgebildet ist, so mit einem oder mehreren beweglich gelagerten Werkzeugen, beispielsweise Maulteilen von Zangen oder Scheren, zu verbinden, daß die Maulteile in gewünschter Weise bewegt werden können. Eine solche Verbindung erfordert einen großen konstruktiven Aufwand. Außerdem kann der Zusammenbau des Instruments sehr aufwendig sein.

Aus der US 6,015,426 ist ein chirurgisches Instrument bekannt, mit einer rotierbaren Verbindungseinrichtung. Ferner ist in der DE 201 21 161 U1 ein endoskopisches Rohrschaftinstrument beschrieben.

Es ist daher Aufgabe der vorliegenden Erfindung, ein chirurgisches Instrument der eingangs beschriebenen Art so zu verbessern, daß das Kraftübertragungsglied auf einfache und sichere Weise mit dem mindestens einen Werkzeug verbunden und im Schaft geführt werden kann.

Diese Aufgabe wird bei einem chirurgischen Instrument der eingangs beschriebenen Art erfindungsgemäß dadurch gelöst, daß die Verdrehsicherung mindestens eine sich in Längsrichtung des Schafts erstreckende Ausnehmung und einen korrespondierenden Vorsprung umfasst, daß die mindestens eine Ausnehmung an der Kupplungsvorrichtung oder am Schaft und daß der Vorsprung am Schaft oder an der Kupplungsvorrichtung angeordnet ist, daß die Ausnehmung eine Abflachung an der Kupplungsvorrichtung umfasst und daß die Kupplungselemente in der Kupplungsstellung durch eine zusätzliche Verbindung form- und/oder kraftschlüssig miteinander verbunden sind.

Ein chirurgisches Instrument der eingangs beschriebenen Art derart weiterzubilden, hat den Vorteil, daß beim Zusammenbau des Instruments keine zusätzliche Verbindung zwischen dem Kraftübertragungsglied und der Kupplungsvorrichtung hergestellt werden muß. Die form- und/oder kraftschlüssige Verbindung zwischen beiden Teilen macht ein zusätzliches Verbindungsverfahren, wie zum Beispiel Kleben oder Löten, überflüssig. Die Kupplungsvorrichtung kann sich nicht vom Kraftübertragungsglied lösen, da die Kupplungsvorrichtung selbst im Schaft geführt wird und so der Schaft als Sicherungselement dient, welcher die Kupplungsvorrichtung und das Kraftübertragungsglied in der Kupplungsstellung hält. Günstigerweise ist die Verdrehsicherung zum Verhindern einer Drehung der Kupplungsvorrichtung um die Längsachse des Schafts vorgesehen. Mit dieser Ausgestaltung wird sichergestellt, daß eine Führung des mindestens einen am distalen Ende des Schafts beweglich gelagerten Werkzeugs relativ zum Schaft unverändert möglich ist, auch dann, wenn das Kraftübertragungsglied um die Längsachse des Schafts verdreht werden sollte. Andersherum ausgedrückt ist ein Verdrehen des beweglich gelagerten Werkzeugs zusammen mit dem Schaft um die Längsachse desselben möglich, ohne daß das Kraftübertragungsglied verdreht wird. Ein besonders einfacher Aufbau des Instruments ergibt sich dadurch, daß die Verdrehsicherung mindestens eine sich in Längsrichtung des Schafts erstreckende Ausnehmung und einen korrespondierenden Vorsprung umfaßt, daß die mindestens eine Ausnehmung an der Kupplungsvorrichtung oder am Schaft und daß der Vorsprung am Schaft oder an der Kupplungsvorrichtung angeordnet ist. Durch das Eintauchen des Vorsprungs in die Ausnehmung wird eine Verdrehung um die Längsachse des Schafts verhindert. Noch einfacher wird der Aufbau des Instruments dadurch, daß die Ausnehmung eine Abflachung an der Kupplungsvorrichtung umfaßt. Damit werden großflächige Anlageflächen an der Kupplungsvorrichtung und am Schaft ausgebildet, welche einfach herzustellen sind und eine optimale Funktion der Verdrehsicherung gewährleisten. Denkbar wäre es, mehrere Kupplungselemente vorzusehen und diese nur über form-und/oder kraftschlüssige Verbindungen am Kraftübertragungsglied zu sichern. Vorteilhaft ist es jedoch, daß mehrere Kupplungselemente vorgesehen sind und daß die Kupplungselemente in der Kupplungsstellung form- und/oder kraftschlüssig miteinander verbunden sind. Durch diese zusätzliche Verbindung wird eine besonders gute Führung der Kupplungselemente im Schaft und eine besonders gute Verbindung der Kupplungselemente mit dem Kraftübertragungsglied erreicht.

Günstig ist es, wenn die Kupplungsvorrichtung mindestens ein einen distalen Endabschnitt des Kraftübertragungsglieds mindestens teilweise umgebendes Kupplungselement aufweist. Auf diese Weise läßt sich die Kupplungsvorrichtung besonders kurz in Richtung der Längsachse ausbilden. Durch das teilweise Umgreifen des Kraftübertragungsglieds durch das mindestens eine Kupplungselement wird die erforderliche form- und/oder kraftschlüssige Verbindung nach Einführung der Kupplungsvorrichtung mit dem Kraftübertragungsglied in den Schaft sicher gehalten.

Vorteilhaft ist es, wenn zwei im wesentlichen halbschalenförmig ausgebildete Kupplungselemente vorgesehen sind. Mit diesen ist es auf einfache Weise möglich, das distale Ende des Kraftübertragungsglieds vollständig zu umschließen, so daß dieses in der Kupplungsstellung sicher mit der Kupplungsvorrichtung verbunden ist. Zudem ist es denkbar, beide Kupplungselemente identisch auszubilden, was den konstruktiven Aufwand zusätzlich minimiert.

Günstig ist es, wenn am Kraftübertragungsglied oder am mindestens einen Kupplungselement mindestens eine Kupplungsaufnahme vorgesehen ist und wenn in der Kupplungsstellung mindestens ein Kupplungsvorsprung des mindestens einen Kupplungselements oder des Schafts in die Kupplungsaufnahme eintaucht. Durch ein Ineingriffbringen der Kupplungsaufnahme und des Kupplungsvorsprungs in der Kupplungsstellung wird eine form- und/oder eine kraftschlüssige Verbindung zwischen dem mindestens einen Kupplungselement und dem Kraftübertragungsglied auf einfache Weise hergestellt.

Ein besonders einfacher Aufbau des Instruments ergibt sich, wenn die Kupplungsaufnahme eine quer zur Längsrichtung offene Nut umfaßt und wenn der Kupplungsvorsprung einen quer zur Längsrichtung abstehenden Vorsprung umfaßt. Durch die Orientierung der Nut und des Kupplungsvorsprungs quer zur Längsrichtung kann auf einfache Weise eine axiale Sicherung des mindestens einen Kupplungselements relativ zum Kraftübertragungsglied hergestellt werden.

Eine besonders einfache Möglichkeit, mehrere Kupplungselemente miteinander zu verbinden, wird gemäß einer bevorzugten Ausführungsform der Erfindung dadurch realisiert, daß jedes der Kupplungselemente jeweils eine zu einer Verzahnung eines anderen Kupplungselementes korrespondierende Verzahnung trägt und daß die Verzahnungen unterschiedlicher Kupplungselemente in der Kupplungsstellung formschlüssig ineinander greifen.

Grundsätzlich könnte die Verzahnung in Richtung auf die Längsachse des Schafts hin weisend ausgebildet sein. Um einen insgesamt besonders kompakten Aufbau des Instruments zu erreichen, ist es jedoch vorteilhaft, wenn die Verzahnung in Umfangsrichtung des Schafts abstehende Vorsprünge umfaßt.

Anstatt oder zusätzlich zu einer Verzahnung kann es vorteilhaft sein, wenn jedes der Kupplungselemente zu der Verzahnung eines anderen Kupplungselements korrespondierende Ausnehmungen aufweist.

Vorteilhaft ist, wenn das Kraftübertragungsglied in der Kupplungsstellung an der Kupplungsvorrichtung um die Längsachse des Schafts drehbar ist. Damit kann die Kupplungsvorrichtung und damit auch das mindestens eine Werkzeug relativ zum Kraftübertragungsglied verdreht werden. Bei einer Verdrehsicherung der Kupplungsvorrichtung relativ zum Schaft kann dann der Schaft mit einem daran beweglich gelagerten Werkzeug relativ zum Kraftübertragungsglied gedreht werden.

Günstig ist es, wenn eine Führungsvorrichtung vorgesehen ist zur Transformation einer axialen Bewegung des Kraftübertragungsglieds in eine Bewegung des Werkzeugs in der Kupplungsstellung. Mit der Führungsvorrichtung kann eine gewünschte Bewegung des mindestens einen Werkzeugs vorgegeben werden.

Besonders einfach wird der Aufbau des Instruments, wenn das mindestens eine Werkzeug um eine quer zur Längsachse des Schafts verlaufende Schwenkachse schwenkbar gelagert ist.

Gemäß einer bevorzugten Ausführungsform der Erfindung kann vorgesehen sein, daß die Führungsvorrichtung eine Führungsausnehmung und einen Führungsvorsprung umfaßt, daß das mindestens eine Werkzeug oder die Kupplungsvorrichtung die Führungsausnehmung aufweist und daß die Kupplungsvorrichtung oder das mindestens eine Werkzeug den in der Kupplungsstellung in der Führungsausnehmung während einer axialen Bewegung des Kraftübertragungsglieds geführten Führungsvorsprung aufweist. Durch diese Ausgestaltung läßt sich das mindestens eine Werkzeug in Folge einer Bewegung der Kupplungsvorrichtung zwangsführen, so daß es in gewünschter Weise bewegt werden kann.

Um in jeder Stellung des mindestens einen Werkzeugs eine sichere Führung zu gewährleisten, kann es günstig sein, wenn jedes der Kupplungselemente einen Führungsvorsprung oder eine Führungsausnehmung aufweist.

Zum Verhindern einer Relativbewegung des Kraftübertragungsglieds und der Kupplungsvorrichtung aufeinander zu, kann es günstig sein, wenn die Kupplungsvorrichtung einen proximalen Anschlag für das distale Ende des Kraftübertragungsglieds aufweist.

Gemäß einer bevorzugten Ausführungsform der Erfindung kann ferner vorgesehen sein, daß die Kupplungsvorrichtung einen proximalen Anschlag für das distale Ende Kraftübertragungsglieds aufweist zum Verhindern einer Relativbewegung des Kraftübertragungsglieds und der Kupplungsvorrichtung voneinander weg.

Alternativ oder zusätzlich kann zum Verhindern einer Relativbewegung des Kraftübertragungsglieds und der Kupplungsvorrichtung voneinander weg und/oder aufeinander zu vorgesehen sein, daß die Kupplungsvorrichtung mindestens ein an einem Rückhalteglied des Kraftübertragungsglieds angreifendes Rückhalteelement umfaßt. Durch das Angreifen des Rückhalteelements am Rückhalteglied in der Kupplungsstellung läßt sich eine axiale Relativbewegung zwischen dem Kraftübertragungsglied und der Kupplungsvorrichtung verhindern.

Um das Instrument als Bipolarinstrument verwenden zu können, kann es vorteilhaft sein, wenn mindestens ein Kupplungselement aus einem elektrisch nichtleitenden Material, insbesondere aus einer Keramik gebildet ist. Auf diese Weise können Teile des Instruments elektrisch voneinander getrennt werden. Das mindestens eine Kupplungselement dient damit zwei Zwecken, einerseits der Verbindung mit dem Kraftübertragungsglied, andererseits der elektrischen Isolierung zwischen zwei stromführenden Teilen.

Die nachfolgende Beschreibung einer bevorzugten Ausführungsform der Erfindung dient im Zusammenhang mit der Zeichnung der näheren Erläuterung. Es zeigen:
- Figur 1:: eine Längsschnittansicht einer erfindungsgemäßen Schere;
- Figur 2:: eine vergrößerte Ansicht des Ausschnitts A in Figur 1;
- Figur 3:: eine Schnittansicht längs Linie 3-3 in Figur 2;
- Figur 4:: eine teilweise durchbrochene Draufsicht auf die Schere in Rich- tung des Pfeils B in Figur 2;
- Figur 5:: eine Längsschnittansicht des vergrößerten Ausschnittsbereichs A ähnlich Figur 2; und
- Figur 6:: eine perspektivische Ansicht eines Kupplungselements.

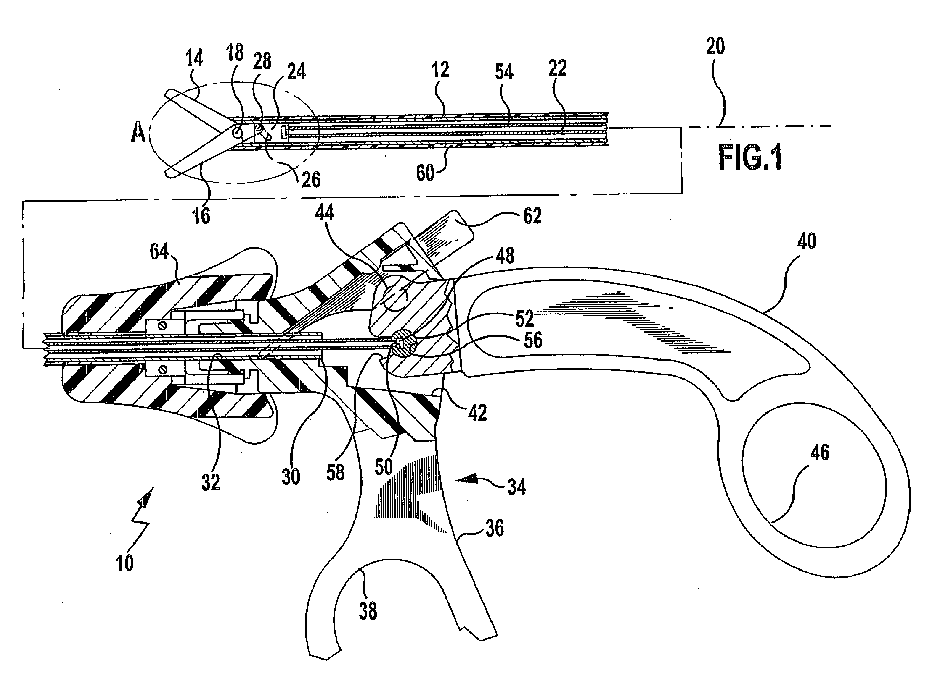

In Figur 1 ist eine Längsschnittansicht einer insgesamt mit dem Bezugszeichen 10 versehenen, als endoskopisches Rohrschaftinstrument ausgebildeten Bipolarschere dargestellt.

Die Bipolarschere 10 umfaßt einen langgestreckten, rohrförmigen Schaft 12, an dessen distalem Ende zwei relativ zueinander verschwenkbare Scherenblätter 14 und 16 auf einem Lagerpin 18 gelagert sind, welcher den Schaft 12 beidseitig und quer zu einer Längsachse 20 des Schafts 12 durchsetzt.

Zum Bewegen der Scherenblätter 14 und 16 ist an einem distalen Ende einer in Richtung der Längsachse 20 im Schaft 12 längsverschieblichen Schub- und Zugstange 22 ein Antriebskörper 24 angeordnet, welcher mit zwei Führungsschlitzen 26 versehen ist, in die quer zur Längsachse 20 von den Scherenblättern 14 und 16 abstehende Lagerzapfen 28 eintauchen und in Folge einer axialen Verschiebung des Antriebskörpers 24 zwangsgeführt werden, wodurch die Scherenblätter 14 und 16 geöffnet beziehungsweise geschossen werden.

An seinem proximalen Ende 30 ist der Schaft 12 in einer Längsbohrung 32 eines feststehenden Griffteils 34 aufgenommen, von dem sich eine feststehende Branche 36 mit einer Fingeröffnung 38 im wesentlichen quer zur Längsachse 20 von dieser weg erstreckt. Am Griffteil 34 ist eine zweite Branche 40 in einer in proximaler Richtung offenen Ausnehmung 42 um einen quer zur Längsachse 20 die Ausnehmung 42 durchsetzenden Lagerbolzen 44 schwenkbar gelagert und weist an ihrem freie Ende eine weitere Fingeröffnung 46 auf.

Ein proximales Ende der Schub- und Zugstange 22 ist mit einem kurzen zylindrischen Kopf 48 versehen, welcher in einer sich einstufig erweiternden Lagernut 50 eines Lagerzylinders 52 formschlüssig eingreift und darin gehalten ist. Aus der Lagernut 50 ragt die Schub- und Zugstange 22 hervor, die mit einer elektrischen Isolierschicht 54 bedeckt ist. Eine Längsachse des Lagerzylinders 52 verläuft quer zur Längsachse 20. Der Lagerzylinder 52 wird an der Branche 40 in der Nähe des Lagerbolzens 44 in einer Lagerbohrung 56 gehalten, welche sich quer zur Längsachse 20 erstreckt und einen sich vom Zentrum der Lagerbohrung 56 in distaler Richtung erweiternden Schlitz 58 umfaßt.

Ebenso wie die Schub- und Zugstange 22 ist auch der Schaft 12 von einer elektrisch isolierenden Schicht 60 umgeben. Sowohl der Schaft 12 als auch die Schub- und Zugstange 22 sind in nicht näher dargestellter Weise mit einem bipolaren Anschluß 62 verbunden, über welchen die Bipolarschere 10 mittels Leitungen an eine elektrische Energieversorgungseinheit angeschlossen werden kann. Sowohl über die Schub- und Zugstange 22 als auch über den Schaft 12 wird jeweils eine elektrische Verbindung zu einem der beiden Scherenblättern 14 und 16 hergestellt, die relativ zueinander isoliert sind. Dies ermöglicht es, zum Koagulieren von Gewebe beispielsweise einen Hochfrequenzstrom über die Scherenblätter 14 und 16 zu leiten und das koagulierte Gewebe im Anschluß an den Koagulationsvorgang zu durchtrennen.

Des weiteren ist ein relativ zum Schaft 12 drehfester Drehknopf 64 vorgesehen, welcher jedoch relativ zum Griffteil verdrehbar ist, so daß das distale Ende der Bipolarschere 10 mit den beiden Scherenblättern 14 und 16 relativ zu den beiden Branchen 36 und 40 um die Längsachse 20 verdreht werden kann.

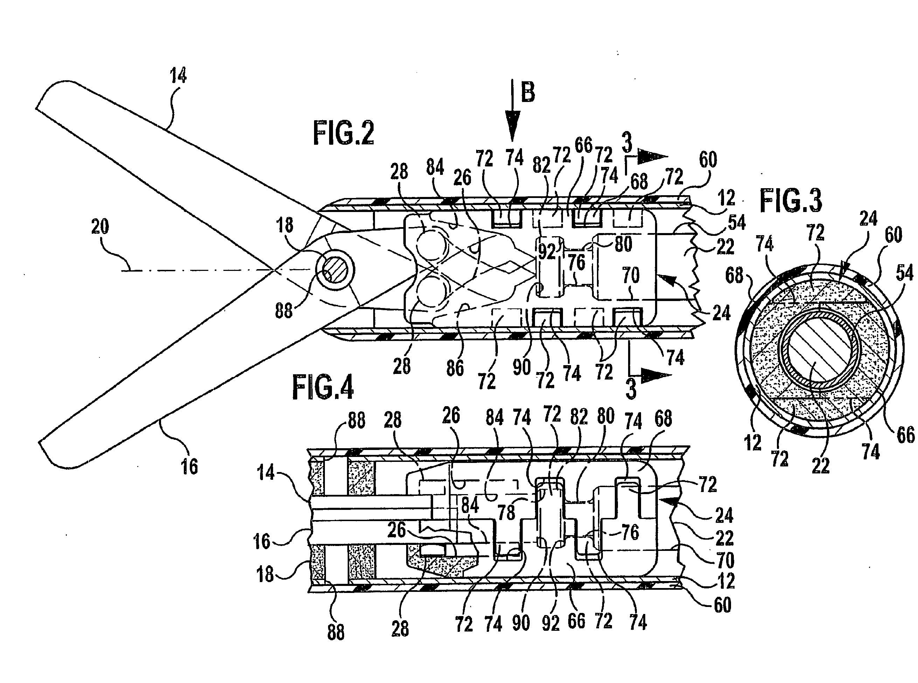

Figur 2 zeigt eine Vergrößerung des Ausschnitts A in Figur 1. Zum Bewegen der beiden Scherenblätter 14 und 16 dient der Antriebskörper 24, welcher aus zwei Halbschalen 66 und 68 zusammengesetzt ist. Die beiden Halbschalen 66 und 68 sind identisch aufgebaut und im wesentlichen in Form eines halben, in Längsrichtung durchtrennten Zylinders ausgebildet. Zum Zusammensetzen weisen beide Halbschalen 66 und 68 jeweils zwei Paare in Umfangsrichtung vorstehender Verbindungszapfen 72 auf, sowie zwei Paare diese aufnehmende Aussparungen 74. Die Aussparungen 74 und die Verbindungszapfen 72 sind abwechselnd entlang des parallel zur Längsachse 20 verlaufenden Randes der Halbschalen 66 und 68 ausgebildet. Im zusammengesetzten Zustand, also in einer Kupplungsstellung, in welcher die beiden Halbschalen 66 und 68 miteinander verbunden sind und die Schub- und Zugstange 22 mit den beiden Scherenblättern 14 und 16 verbinden, bilden die beiden Halbschalen 66 und 68 proximalseitig eine Stangenaufnahme 70 in Form einer Bohrung aus. Diese verjüngt sich einstufig auf einem kurzen Abschnitt und erweitert sich dann wieder im Durchmesser einstufig, so daß ein Ringvorsprung 76 zwischen der Stangenaufnahme 70 und einer Ringnut 78 gebildet wird.

Ein distales Ende der Schub- und Zugstange 22 weist eine zum Ringvorsprung 76 korrespondierende Ringnut 80 und einen sich daran anschließenden zylinderförmigen Kopf 82 auf, welcher korrespondierend zur Ringnut 78 ausgebildet ist.

Distalseitig sind beide Halbschalen 66 und 68 mit im wesentlichen V-förmigen, in distaler Richtung offenen Ausnehmungen 84 und 86 versehen, wobei in jeder Ausnehmung in Richtung auf den Schaft 12 jeweils einer der beiden Führungsschlitze 26 eingearbeitet ist. Die beiden Führungsschlitze 26 bilden eine quer zur Längsachse 20 und auf diese hin offene Nut, in welche die Lagerzapfen 28 der Scherenblätter 14 und 16 jeweils quer zur Längsachse 20 und von dieser weg weisend eintauchen. Die Führungsschlitze 26 sind leicht gekrümmt. Zum Zusammenbauen des vorderen Endes der Bipolarschere 10 werden zunächst die beiden Scherenblätter 14 und 16 mit ihren Lagerzapfen 28 in die jeweiligen Führungsschlitze 26 eingesetzt und die Schub- und Zugstange 22 mit ihrem Kopf 82 in die Ringnut 78 eingelegt. Zwischen die beiden Scherenblätter 14 und 16 ist ferner eine in den Figuren nicht dargestellte Kontaktfolie oder ein Kontaktblättchen eingelegt, welche am Kopf 82 der Schub- und Zugstange 22 anliegen. Eines der beiden Scherenblätter 14 und 16 ist relativ zum Lagerpin 18 elektrisch isoliert, zum Beispiel durch eine aus einem nichtleitenden Material, beispielsweise einer Keramik hergestellte, den Lagerpin 18 umgebende Lagerbüchse. Auf diese Weise ist das eine Scherenblatt auch gegenüber dem anderen Scherenblatt isoliert, so daß eine elektrische Verbindung der Kontaktfolie nur zu einem Scherenblatt hergestellt wird. So ist es möglich, vom bipolaren Anschluß 62 einen elektrischen Strom über die Schub- und Zugstange 22 und die Kontaktfolie zu einem der beiden Scherenblätter 14 und 16 und über das andere der beiden Scherenblätter 14 und 16, den Lagerpin 18 und den Schaft 12 wieder zurück zum bipolaren Anschluß 62 zu leiten.

Anschließend werden die beiden Halbschalen 66 und 68 quer zur Längsachse 22 zusammengeschoben, so daß jeder Verbindungszapfen 72 der Halbschalen 66 in eine Aussparung 74 der Halbschale 68 eintaucht und umgekehrt. Die Halbschalen 66 und 68 werden nicht miteinander verklebt oder auf andere Weise unlösbar miteinander verbunden.

Nach dem beschriebenen Zusammenfügen der Teile wird die Schub- und Zugstange 22 von distal her kommend in den Schaft 12 eingeführt, bis der Schaft 12 die Halbschalen 66 und 68 umgibt. Sobald die Halbschalen 66 und 68 in den Schaft 12 eingeführt sind, sind die Schub- und Zugstange 22 und die Scherenblättern 14 in axialer Richtung unlösbar miteinander verbunden, denn der Schaft 12 sichert die Halbschalen 66 und 68 gegen ein Lösen von der Schub- und Zugstange 22. Abschließend wird der Lagerpin 18 durch quer zur Längsachse verlaufende Bohrungen 88 im Schaft 12 hindurchgesteckt, wodurch die Scherenblätter 14 und 16 am Schaft 12 festgelegt werden, wodurch aufgrund einer Bewegung der Schub- und Zugstange in axialer Richtung und durch Führung der Lagerzapfen 28 in den Führungsschlitzen 26 nur noch eine Schwenkbewegung der Scherenblätter 14 und 16 aufeinander zu beziehungsweise voneinander weg möglich ist.

In Figur 3 ist eine Querschnittsansicht dargestellt, in welcher das formschlüssige Ineinandergreifen der Verbindungszapfen 72 der Halbschale 66 in eine Aussparung 74 der Halbschale 68 und umgekehrt dargestellt ist. Ferner ist zu erkennen, daß die beiden Halbschalen 66 und 68 das Innere des Schafts 12 praktisch vollständig ausfüllen und das distale Ende der Schub- und Zugstange 22 vollständig umschließen. Ein Lösen der Verbindung zwischen den beiden, den Antriebskörper 24 bildenden Halbschalen 66 und 68 von der Schub- und Zugstange 22 ist in der in den Figuren 2 bis 5 dargestellten Kupplungsstellung nicht möglich. Hierfür müßte der Antriebskörper 24 aus dem distalen Ende des Schafts herausgeschoben werden.

In der in der Figur 4 dargestellten Draufsicht ist zu erkennen, wie die Verbindungszapfen 72 eine Art Verzahnung bilden, welche in die korrespondierenden Aussparungen 74 des jeweils anderen Kupplungselements, welches in Form einer Halbschale 66 beziehungsweise 68 ausgebildet ist, eingreift.

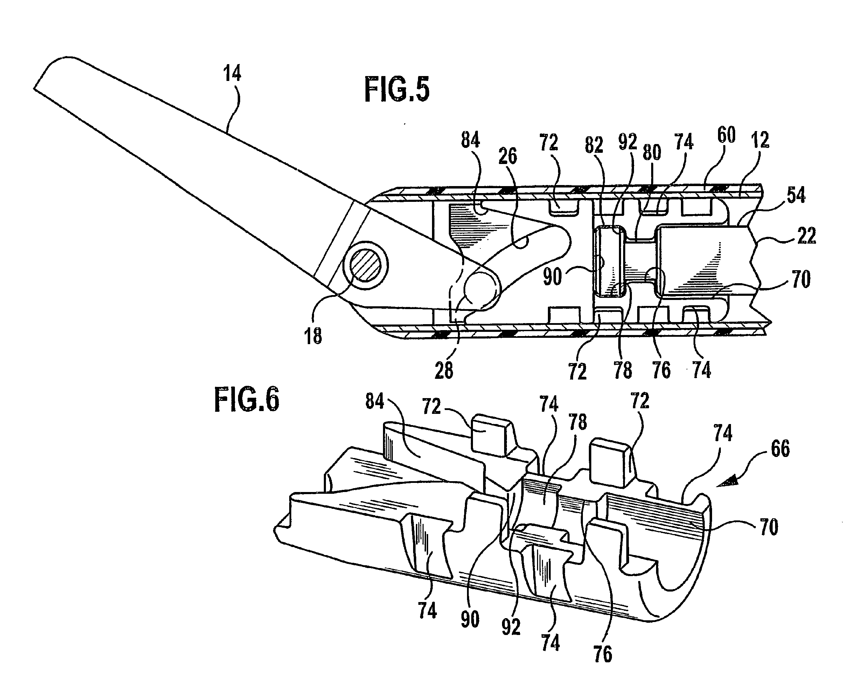

In den Figuren 5 und 6 ist besonders gut zu erkennen, daß eine distale Seitenwand der Ringnut 78 einen Anschlag 90 bildet, welcher eine Bewegung der Schub- und Zugstange 22 in distaler Richtung begrenzt. In prinzipiell gleicher Weise wird eine Bewegung der Schub- und Zugstange in proximaler Richtung begrenzt durch eine parallel zum Anschlag 90 verlaufende Ringkante 92, welche eine proximale Seitenwand der Ringnut 78 bildet.

In Figur 6 ist exemplarisch eine Halbschale 66 dargestellt, die identisch mit der Halbschale 68 ausgebildet ist. Die Halbschalen 66 und 68 können entweder aus einem Metall oder aus einer Keramik hergestellt werden.

## Patentansprüche

1. Chirurgisches Instrument (10) mit einem eine Längsachse (20) definierenden Schaft (12), mit mindestens einem an einem distalen Ende des Schafts (12) beweglich gelagerten Werkzeug (14, 16) und mit einem im Schaft (12) axial beweglich gelagerten Kraftübertragungsglied (22) zum Übertragen von am proximalen Ende des Instruments (10) auf das Kraftübertragungsglied (22) zum Bewegen des Werkzeugs (14, 16) ausgeübten Zug- und/oder Schubkräften, wobei eine Kupplungsvorrichtung (24) vorgesehen ist zum axialen Verbinden des Kraftübertragungsglieds (22) mit dem mindestens einen Werkzeug (14, 16) in einer Kupplungsstellung, wobei die Kupplungsvorrichtung (24) in der Kupplungsstellung form- und/oder kraftschlüssig mit dem Kraftübertragungsglied (22) verbunden ist, wobei die Kupplungsvorrichtung (24) im Schaft (12) axial geführt ist, wobei eine Verdrehsicherung vorgesehen ist zum Verhindern einer Drehung der Kupplungsvorrichtung (24) um die Längsachse (20) des Schafts (12) und wobei mehrere Kupplungselemente (66, 68) vorgesehen sind, **dadurch gekennzeichnet, daß** die Verdrehsicherung mindestens eine sich in Längsrichtung (20) des Schafts (12) erstreckende Ausnehmung und einen korrespondierenden Vorsprung umfaßt, daß die mindestens eine Ausnehmung an der Kupplungsvorrichtung (24) oder am Schaft (12) und daß der Vorsprung am Schaft (12) oder an der Kupplungsvorrichtung (24) angeordnet ist, daß die Ausnehmung eine Abflachung an der Kupplungsvorrichtung (24) umfaßt und daß die Kupplungselemente (66, 68) in der Kupplungsstellung durch eine zusätzliche Verbindung form- und/oder kraftschlüssig miteinander verbunden sind.

2. Instrument nach Anspruch 1, **dadurch gekennzeichnet, daß** die Kupplungsvorrichtung (24) mindestens ein einen distalen Endabschnitt (80, 82) des Kraftübertragungsglieds (22) mindestens teilweise umgebendes Kupplungselement (66, 68) aufweist.

3. Instrument nach Anspruch 2, **dadurch gekennzeichnet, daß** zwei im wesentlichen halbschalenförmig ausgebildete Kupplungselemente (66, 68) vorgesehen sind.

4. Instrument nach einem der voranstehenden Ansprüche, **dadurch gekennzeichnet, daß** am Kraftübertragungsglied (22) oder am mindestens einen
Kupplungselement (66, 68) mindestens eine Kupplungsaufnahme (80; 78) vorgesehen ist und daß in der Kupplungsstellung mindestens ein Kupplungsvorsprung (76; 82) des mindestens einen Kupplungselements (66, 68) oder des Kraftübertragungsglieds (22) in die Kupplungsaufnahme (80; 78) eintaucht.

5. Instrument nach Anspruch 4, **dadurch gekennzeichnet, daß** die Kupplungsaufnahme (80; 78) eine quer zur Längsrichtung (20) offene Nut umfaßt und daß der Kupplungsvorsprung (76; 82) einen quer zur Längsrichtung (20) abstehenden Vorsprung umfaßt.

6. Instrument nach einem der voranstehenden Ansprüche, **dadurch gekennzeichnet, daß** jedes der Kupplungselemente (66, 68) jeweils eine zu einer Verzahnung (72, 74) eines anderen Kupplungselements (66, 68)
korrespondierende Verzahnung (72, 74) trägt und daß die Verzahnungen (72, 74) unterschiedlicher Kupplungselemente (66, 68) in der Kupplungsstellung formschlüssig ineinandergreifen.

7. Instrument nach Anspruch 6, **dadurch gekennzeichnet, daß** die Verzahnung (72, 74) in Umfangsrichtung des Schafts (12) abstehende Vorsprünge (72) umfaßt.

8. Instrument nach einem der Ansprüche 6 oder 7, **dadurch gekennzeichnet, daß** jedes der Kupplungselemente (66, 68) zu der Verzahnung (72,74) eines anderen Kupplungselements (66, 68) korrespondierende Ausnehmungen (74) aufweist.

9. Instrument nach einem der voranstehenden Ansprüche, **dadurch gekennzeichnet, daß** das Kraftübertragungsglied (22) in der Kupplungsstellung an der Kupplungsvorrichtung (24) um die Längsachse (20) des Schafts (12) drehbar ist.

10. Instrument nach einem der voranstehenden Ansprüche, **dadurch gekennzeichnet, daß** eine Führungsvorrichtung (26, 28) vorgesehen ist zur Transformation einer axialen Bewegung des Kraftübertragungsglieds (22) in eine Bewegung des Werkzeugs (14, 16) in der Kupplungsstellung.

11. Instrument nach einem der voranstehenden Ansprüche, **dadurch gekennzeichnet, daß** das mindestens eine Werkzeug (14, 16) um eine quer zur Längsachse (20) des Schafts (12) verlaufende Schwenkachse schwenkbar gelagert ist.

12. Instrument nach einem der Ansprüche 10 oder 11, **dadurch gekennzeichnet, daß** die Führungsvorrichtung (26, 28) eine Führungsausnehmung (26) und einen Führungsvorsprung (28) umfaßt, daß das mindestens eine Werkzeug (14, 16) oder die Kupplungsvorrichtung (24) die Führungsausnehmung (26) aufweist und daß die Kupplungsvorrichtung (24) oder das mindestens eine Werkzeug (14, 16) den in der Kupplungsstellung in der Führungsausnehmung (26) während einer axialen Belegung des Kraftübertragungsgliedes (22) geführten Führungsvorsprung (28) aufweist.

13. Instrument nach Anspruch 12, **dadurch gekennzeichnet, daß** der Führungsvorsprung (28) im wesentlichen parallel zur Schwenkachse absteht.

14. Instrument nach einem der Ansprüche 3 bis 13, **dadurch gekennzeichnet, daß** jedes der Kupplungselemente (66,68) einen Führungsvorsprung (28) oder eine Führungsausnehmung (26) aufweist.

15. Instrument nach einem der voranstehenden Ansprüche, **dadurch gekennzeichnet, daß** die Kupplungsvorrichtung (24) einen proximalen Anschlag (90) für das distale Ende des Kraftübertragungsglieds (22) aufweist zum Verhindern einer Relativbewegung des Kraftübertragungsglieds (22) und der Kupplungsvorrichtung (24) aufeinander zu.

16. Instrument nach einem der voranstehenden Ansprüche, **dadurch gekennzeichnet, daß** die Kupplungsvorrichtung (24) einen proximalen Anschlag (90) für das distale Ende des Kraftübertragungsglied (22) aufweist zum Verhindern einer Relativbewegung des Kraftübertragungsglieds (22) und der Kupplungsvorrichtung (24) voneinander weg.

17. Instrument nach einem der voranstehenden Ansprüche, **dadurch gekennzeichnet, daß** die Kupplungsvorrichtung (24) mindestens ein an einem Rückhalteglied (80) des Kraftübertragungsgliedes (22) angreifendes Rückhalteelement (76) umfaßt zum Verhindern einer Relativbewegung des Kraftübertragungsglieds (22) und der Kupplungsvorrichtung (24) voneinander weg und/oder aufeinander zu.

18. Instrument nach einem der voranstehenden Ansprüche, **dadurch gekennzeichnet, daß** mindestens ein Kupplungselement (66, 68) aus einem elektrisch nichtleitenden Material, insbesondere aus einer Keramik gebildet ist.

## Claims

1. Surgical instrument (10) with a shaft (12) defining a longitudinal axis (20), with at least one tool (14, 16) mounted for movement at a distal end of the shaft (12) and with a force transmission member (22) mounted for axial movement in the shaft (12) for transmitting push and/or pull forces applied at the proximal end of the instrument (10) onto the force transmission member (22) in order to move the tool (14, 16), a coupling device (24) being provided for axially connecting the force transmission member (22) to the at least one tool (14, 16) in a coupled position, the coupling device (24) being connected with positive locking and/or force locking to the force transmission member (22) in the coupled position, the coupling device (24) being axially guided in the shaft (12), a lock against rotation being provided for preventing rotation of the coupling device (24) about the longitudinal axis (20) of the shaft (12), and a plurality of coupling elements (66, 68) being provided, **characterized in that** the lock against rotation comprises at least one recess extending in the longitudinal direction (20) of the shaft (12) and a corresponding projection, **in that** the at least one recess is arranged on the coupling device (24) or on the shaft (12) and the projection on the shaft (12) or on the coupling device (24), **in that** the recess has a flattening on the coupling device (24), and **in that** the coupling elements (66, 68), in the coupled position, are connected to each other with positive locking and/or force locking by an additional connection.

2. Instrument in accordance with claim 1, **characterized in that** the coupling device (24) comprises at least one coupling element (66, 68) surrounding at least partially a distal end portion (80, 82) of the force transmission member (22).

3. Instrument in accordance with claim 2, **characterized in that** two coupling elements (66, 68) of substantially half-shell-shaped configuration are provided.

4. Instrument in accordance with any one of the preceding claims, **characterized in that** at least one coupling receptacle (80; 78) is provided on the force transmission member (22) or on the at least one coupling element (66, 68), and **in that**, in the coupled position, at least one coupling projection (76; 82) of the at least one coupling element (66, 68) or of the force transmission member (22) enters the coupling receptacle (80; 78).

5. Instrument in accordance with claim 4, **characterized in that** the coupling receptacle (80; 78) comprises a groove which is open transversely to the longitudinal direction (20), and **in that** the coupling projection (76; 82) comprises a projection protruding transversely to the longitudinal direction (20).

6. Instrument in accordance with any one of the preceding claims, **characterized in that** each of the coupling elements (66, 68) carries a toothing (72, 74) corresponding to a toothing (72, 74) of another coupling element (66, 68), and **in that** the toothings (72, 74) of different coupling elements (66, 68) engage one another with positive locking in the coupled position.

7. Instrument in accordance with claim 6, **characterized in that** the toothing (72, 74) comprises projections (72) protruding in the circumferential direction of the shaft (12).

8. Instrument in accordance with claim 6 or 7, **characterized in that** each of the coupling elements (66, 68) comprises recesses (74) corresponding to the toothing (72, 74) of another coupling element (66, 68).

9. Instrument in accordance with any one of the preceding claims, **characterized in that** the force transmission member (22) in the coupled position is rotatable on the coupling device (24) about the longitudinal axis (20) of the shaft (12).

10. Instrument in accordance with any one of the preceding claims, **characterized in that** a guide device (26, 28) is provided for transformation of an axial movement of the force transmission member (22) into a movement of the tool (14, 16) in the coupled position.

11. Instrument in accordance with any one of the preceding claims, **characterized in that** the at least one tool (14, 16) is mounted for pivotal movement about a pivot axis extending transversely to the longitudinal axis (20) of the shaft (12).

12. Instrument in accordance with claim 10 or 11, **characterized in that** the guide device (26, 28) comprises a guide recess (26) and a guide projection (28), **in that** the at least one tool (14, 16) or the coupling device (24) comprises the guide recess (26), and **in that** the coupling device (24) or the at least one tool (14, 16) comprises the guide projection (28) guided, in the coupled position, in the guide recess (26) during axial movement of the force transmission member (22).

13. Instrument in accordance with claim 12, **characterized in that** the guide projection (28) protrudes substantially parallel to the pivot axis.

14. Instrument in accordance with any one of claims 3 to 13, **characterized in that** each of the coupling elements (66, 68) comprises a guide projection (28) or a guide recess (26).

15. Instrument in accordance with any one of the preceding claims, **characterized in that** the coupling device (24) comprises a proximal stop (90) for the distal end of the force transmission member (22) for preventing relative movement of the force transmission member (22) and the coupling device (24) towards each other.

16. Instrument in accordance with any one of the preceding claims, **characterized in that** the coupling device (24) comprises a proximal stop (90) for the distal end of the force transmission member (22) for preventing relative movement of the force transmission member (22) and the coupling device (24) away from each other.

17. Instrument in accordance with any one of the preceding claims, **characterized in that** the coupling device (24) comprises at least one retaining element (76) engaging a retaining member (80) of the force transmission member (22) for preventing relative movement of the force transmission member (22) and the coupling device (24) away from each other and/or towards each other.

18. Instrument in accordance with any one of the preceding claims, **characterized in that** at least one coupling element (66, 68) is formed from an electrically non-conductive material, in particular, from a ceramic material.

## Revendications

1. Instrument chirurgical (10) comprenant un corps allongé (12) qui définit un axe longitudinal (20), comprenant également au moins un outil (14, 16) monté mobile à une extrémité distale du corps allongé (12), et un organe de transmission de force (22), qui est monté axialement mobile dans le corps allongé (12) et est destiné à la transmission de forces de traction et/ou de poussée exercées sur l'organe de transmission de force (22) à l'extrémité proximale de l'instrument (10), pour produire un mouvement de l'outil (14, 16), instrument dans lequel est prévu un dispositif d'accouplement (24) pour assurer la liaison axiale de l'organe de transmission de force (22) avec ledit au moins un outil (14, 16), dans une position d'accouplement,
dans lequel le dispositif d'accouplement (24), en position d'accouplement, est relié par complémentarité de formes et/ou par adhérence à l'organe de transmission de force (22),
dans lequel le dispositif d'accouplement (24) est guidé axialement dans le corps allongé (12),
dans lequel il est prévu un système d'arrêt de rotation relative destiné à empêcher une rotation du dispositif d'accouplement (24) autour de l'axe longitudinal (20) du corps allongé (12),
et dans lequel sont prévus plusieurs éléments d'accouplement (66, 68),
**caractérisé en ce que** le système d'arrêt de rotation relative comprend au moins un évidement, qui s'étend dans la direction longitudinale (20) du corps allongé (12), et une protubérance correspondante, **en ce que** ledit au moins un évidement est agencé sur le dispositif d'accouplement (24) ou sur le corps allongé (12), et **en ce que** la protubérance est agencée sur le corps allongé (12) ou sur le dispositif d'accouplement (24), **en ce que** l'évidement comprend un méplat sur le dispositif d'accouplement (24), et **en ce que** les éléments d'accouplement (66, 68) sont, dans la position d'accouplement, reliés mutuellement par une liaison supplémentaire par complémentarité de formes et/ou par adhérence.

2. Instrument selon la revendication 1, **caractérisé en ce que** le dispositif d'accouplement (24) présente au moins un élément d'accouplement (66, 68) entourant au moins partiellement un tronçon d'extrémité distal (80, 82) de l'organe de transmission de force (22).

3. Instrument selon la revendication 2, **caractérisé en ce que** sont prévus deux éléments d'accouplement (66, 68) d'une configuration sensiblement en forme de demi-coque.

4. Instrument selon l'une des revendications précédentes, **caractérisé en ce que** sur l'organe de transmission de force (22) ou sur ledit au moins un élément d'accouplement (66, 68) est prévu au moins un logement d'accouplement (80 ; 78), et **en ce que** dans la position d'accouplement, au moins une protubérance d'accouplement (76 ; 82) dudit au moins un élément d'accouplement (66, 68) ou de l'organe de transmission de force (22) s'engage dans le logement d'accouplement (80 ; 78).

5. Instrument selon la revendication 4, **caractérisé en ce que** le logement d'accouplement (80 ; 78) comprend une rainure ouverte transversalement à la direction longitudinale (20), et **en ce que** la protubérance d'accouplement (76 ; 82) comprend une protubérance en saillie transversalement à la direction longitudinale (20).

6. Instrument selon l'une des revendications précédentes, **caractérisé en ce que** chacun des éléments d'accouplement (66, 68) porte respectivement une denture (72, 74) en correspondance avec une denture (72, 74) d'un autre élément d'accouplement (66, 68), et **en ce que** les dentures (72, 74) d'éléments d'accouplement (66, 68) différents sont en prise réciproque par complémentarité de formes, dans la position d'accouplement.

7. Instrument selon la revendication 6, **caractérisé en ce que** la denture (72, 74) comprend des protubérances (72) faisant saillie dans la direction périphérique du corps allongé (12).

8. Instrument selon l'une des revendications 6 ou 7, **caractérisé en ce que** chacun des éléments d'accouplement (66, 68) présente des évidements (74) en correspondance avec la denture (72, 74) d'un autre élément d'accouplement (66, 68).

9. Instrument selon l'une des revendications précédentes, **caractérisé en ce que** l'organe de transmission de force (22), dans la position d'accouplement, peut tourner au niveau du dispositif d'accouplement (24), autour de l'axe longitudinal (20) du corps allongé (12).

10. Instrument selon l'une des revendications précédentes, **caractérisé en ce qu'**il est prévu un dispositif de guidage (26, 28) pour la transformation d'un mouvement axial de l'organe de transmission de force (22) en un mouvement de l'outil (14, 16), dans la position d'accouplement.

11. Instrument selon l'une des revendications précédentes, **caractérisé en ce que** ledit au moins un outil (14, 16) est monté de manière à pouvoir pivoter autour d'un axe de pivotement, qui s'étend transversalement à l'axe longitudinal (20) du corps allongé (12).

12. Instrument selon l'une des revendications 10 ou 11, **caractérisé en ce que** le dispositif de guidage (26, 28) comprend un évidement de guidage (26) et une protubérance de guidage (28), **en ce que** ledit au moins un outil (14, 16) ou le dispositif d'accouplement (24) présente l'évidement de guidage (26), et **en ce que** le dispositif d'accouplement (24) ou ledit au moins un outil (14, 16) présente la protubérance de guidage (28), qui, dans la position d'accouplement, est guidée dans l'évidement de guidage (26) pendant un mouvement axial de l'organe de transmission de force (22).

13. Instrument selon la revendication 12, **caractérisé en ce que** la protubérance de guidage (28) fait saillie sensiblement de manière parallèle à l'axe de pivotement.

14. Instrument selon l'une des revendications 3 à 13, **caractérisé en ce que** chacun des éléments d'accouplement (66, 68) présente une protubérance de guidage (28) ou un évidement de guidage (26).

15. Instrument selon l'une des revendications précédentes, **caractérisé en ce que** le dispositif d'accouplement (24) présente une butée proximale (90) pour l'extrémité distale de l'organe de transmission de force (22), en vue d'empêcher un mouvement relatif de l'organe de transmission de force (22) et du dispositif d'accouplement (24) l'un vers l'autre.

16. Instrument selon l'une des revendications précédentes, **caractérisé en ce que** le dispositif d'accouplement (24) présente une butée proximale (90) pour l'extrémité distale de l'organe de transmission de force (22), en vue d'empêcher un mouvement relatif d'éloignement l'un de l'autre de l'organe de transmission de force (22) et du dispositif d'accouplement (24).

17. Instrument selon l'une des revendications précédentes, **caractérisé en ce que** le dispositif d'accouplement (24) comprend au moins un élément de retenue (76), qui interagit avec un organe de retenue (80) de l'organe de transmission de force (22), en vue d'empêcher un mouvement relatif de l'organe de transmission de force (22) et du dispositif d'accouplement (24) de l'un vers l'autre et/ou d'éloignement l'un de l'autre.

18. Instrument selon l'une des revendications précédentes, **caractérisé en ce qu'**au moins un élément d'accouplement (66, 68) est réalisé en un matériau électriquement non conducteur, notamment en une céramique.
